# EUROPEAN PATENT APPLICATION

(11) **EP 4 468 204 A1**
(43) Date of publication of application: **27.11.2024**
(21) Application number: 23175672.7
(22) Date of filing: 26.05.2023
(51) Int. Cl.: G06N 3/0475, G06N 3/045, G06N 3/0895

(54) **AUTOMATIC OPTIMIZATION OF PARAMETERS OF AN IMAGE PROCESSING CHAIN**

(71) Applicant: Siemens Healthineers AG, 91301 Forchheim (DE)
(72) Inventor: Eckert, Dominik, 90762 Fürth (DE); Kappler, Steffen, 91090 Effeltrich (DE); Ritschl, Ludwig, 96155 Buttenheim (DE)
(74) Representative: Siemens Healthineers Patent Attorneys

(57) **Abstract**

A method for AI-assisted generating an adapted medical image processing chain (A-PC) is described. The method comprises the step of receiving medical image data (RP-MI, T-MI) representing only a piece of information of a complete matching pair of medical image data related to a target flavor, wherein another piece of information (SRP-MI, ST-MI, FRD, SRD) of the complete matching pair is missing. The method also comprises the automatic step of generating an estimated medical image (E-MI) by applying a medical image processing chain (PC) to a raw projection medical image (RP-MI) that is related to the received medical image data (RP-MI, T-MI). Further, the method comprises the step of determining a result (ROC) of a comparison based on the estimated medical image (E-MI) and a target medical image (T-MI) which is also related to the received medical image data. The method furthermore comprises the step of generating an adapted medical image processing chain (A-PC) by adapting the medical image processing chain (PC) based on the result (ROC) of the comparison. The missing piece of information (SRP-MI, ST-MI, FRD, SRD) is generated based on the received medical image data (RP-MI, T-MI) using an AI-based model (AI-M1, AI-M2, AI-M3). Besides, a method for generating a trained AI-based model (AI-M1, AI-M2, AI-M3) for estimating a missing piece of information (SRP-MI, ST-MI, FRD, SRD) of a complete matching pair of medical image data related to a target flavor is described. Also an adaption device (80) is also provided.

## Description

The invention relates to a method for AI-assisted generating an adapted medical image processing chain. Further, the invention relates to a method for generating a trained AI-based model for estimating a missing piece of information of a complete matching pair of medical image data related to a target flavour. Furthermore, the invention concerns an adaption device.

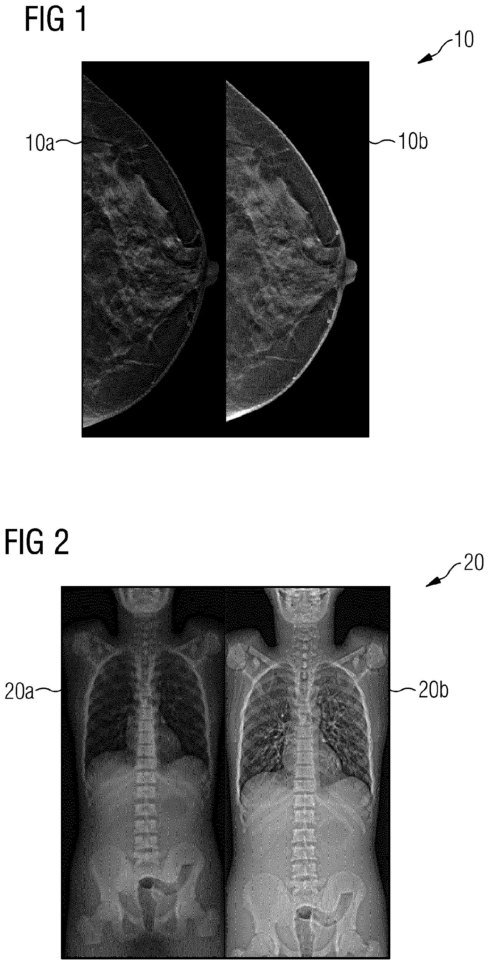

X-rays have different image impressions called flavors. These flavors depend on the hardware used to record them as well as on the operation principle and the parameter set of the applied reconstruction and the processing algorithm. The influence of the chosen recording system on the X-ray image impression can be observed in FIG 1 and FIG 2. FIG 1 shows an example of two reconstructed X-ray images of the same breast, but recorded with a different parameter set of the post processing algorithm. The same physical phantom scanned with two different X-ray machines generating medical image with different flavors, i.e. impressions, is presented in FIG 2.

Radiologists are used to a particular flavor. Hence, changing or working with different image impression leads to an increase in the reading time and a decrease of the reliability of the diagnosis. This is especially problematic, if different X-ray images with different flavors must be compared against each other. However, there are several reasons, which make the use or exchange of various x-ray systems necessary: An X-ray system exchange or update with more recent technology increases the image quality and consequently the reliability of the diagnosis, an old X-ray machine must be replaced due to a defect, or a radiologist changes the working side.

When a new system or software version is installed at a customer site, service or image quality specialists adjust post processing parameters by hand together with the customer, using some example cases from the local PACS (PACS is an acronym for Picture Archiving and Communication System). This is very time consuming, and the results are not always satisfying.

For adapting an image processing chain, a pair of images is necessary, wherein a first image comprises an unprocessed raw projection medical image and a second image comprises a processed target medical image including the desired flavor and both types of images depict the same image content. However, typically, there is only one set of processed images from the radiologist with the desired flavor, but no matching unprocessed raw projection medical image. The absence of such a complete matching pair prevents the adaption of an image processing chain in which raw projections are fed into the processing chain and in which the so obtained processed medical images are compared against their corresponding matching target medical images comprising the desired flavor.

Hence, a general problem underlying to the invention is to adapt characteristics of processed X-ray images to desired image characteristics, in particular the desired flavor.

The before-mentioned problem is solved by a method for AI-assisted generating an adapted medical image processing chain according to claim 1, by a method for generating a trained AI-based model for estimating a missing piece of information of a complete matching pair of medical image data related to a target flavor according to claim 10 and by an adaption device according to claim 13.

By the method for AI-assisted generating an adapted medical image processing chain (AI is an acronym for "artificial intelligence"), medical image data representing only a piece of information of a complete matching pair of medical image data related to a target flavor are received, wherein another piece of information of the complete matching pair is missing. A complete matching pair comprises a pair of a raw projection medical image and a target medical image. A complete matching pair of medical image data means that both images of the "matching pair" include the same image content and the target medical image includes the target flavor. A piece of information means that the received medical image data are incomplete and do not represent the ideal complete matching pair. Hence, there is a lack of information at the beginning of the method according to the invention, which has to be compensated for achieving a correct adaption of the medical image processing chain to a target flavor.

As later described in detail, an "incomplete matching pair" preferably means that the situation may arise that only one of the following sets of images are available:
- only a raw projection medical image,
- only a target medical image,
- a raw projection medical image and a target medical image, wherein the content of the raw projection medical image is not the same as the content of the target medical image.

Based on the available image data measurements have to be performed to compensate the lack of information. As later described, in one variant, an AI-based model compensates the lack of information about a target flavor, in another variant, an AI-based model compensates the lack of information about raw projection medical image data corresponding to the target medical image and in a third variant, an AI-based model compensates the lack of paired medical images with the same content.

Firstly, an estimated medical image is automatically generated by applying the medical image processing chain to a raw projection medical image. A medical image processing chain comprises a sequence of filter units for processing a raw projection medical image. The characteristics of each of the filter units can be modified by altering parameter values of parameters assigned to each of these filter units. Parameters of an image chain relate to the windowing, which is related to the division of grey value intervals. Further, parameters can also be related to the presentation of the background. Furthermore, parameters can also concern cutting or resampling of value ranges, the use of color look-up tables or filters, and the adjustment of the bit depth or sampling depth.

A raw projection medical image is an unprocessed medical image as it directly results from the X-ray projection and image recordation without any post-processing.

For the object of adapting the resulting flavor of an image processing chain to a desired type of flavor, an image processing chain has to be adapted. For that object, the medical image processing chain is applied to the raw projection medical image. A flavor can be defined by a set of parameters. Such a set of parameters preferably comprises at least one of the following types of parameters: the sharpness of the image in high frequencies, low contrasts, dynamic behaviour, in particular windowing for specific organs like abdomen or lung, dynamic range compression, tone mapping.

Tone mapping, tone reproduction or dynamic range compression are synonymous terms that describe the compression of the dynamic range of high-contrast images (high dynamic range images), i.e. digital images with a high brightness range. With tone mapping, the contrast range of a high-contrast image is reduced in order to be able to display it on conventional output devices.

Further, a result of a comparison based on the estimated medical image and a target medical image is automatically determined. For the comparison and adaption, different actions have to be taken to compensate the lack of information.

In particular, for comparison the style of different images, different options appear. For instance, local Laplacian filters exist for transferring the style from one image to another. These filters have originally been investigated for photographical images and are for example part of a photo editing software. These filters can be modified in a way that the remap functions of each layer of the Laplacian pyramid reflect the style difference of two images: The closer the remap functions are on the diagonal, the greater the similarity between the two medical images is. This can be reflected by one number using the mean difference of the areas under curve. Another possibility would be to analyze the histograms of different frequency bands of the two medical images. For instance, a histogram matching technique was introduced to standardize image impressions as preprocessing technique for increasing the robustness of machine learning algorithms. Accordingly, a measure of similarity can be derived by comparing the frequency-band histograms (e.g., mean and standard deviation) of the two medical images.

As later discussed in detail, the type of comparison depends on the type of the medical image data available for the comparison.

Based on the result of the comparison, an adapted medical image processing chain is generated by adapting the medical image processing chain. The adaption based on the comparison, i.e. the similarity measure, is preferably used within an optimization procedure, e.g. a gradient descent method or a Newton method for an iterative adjustment of parameters of the medical image processing chain.

Advantageously, a medical image processing chain is automatically adapted for generating medical image data with a desired flavour without involving a human expert having to intervene in the adaption process based on an incomplete matching pair of medical images. By adapting a medical image processing chain of a new medical imaging system, in particular an X-ray imaging system, to a flavor of an old medical imaging system used by radiologists before, a more precise and faster reading of the medical images on the new medical imaging system by the radiologists is achieved.

As later discussed in detail, for compensating a lack of an available complete matching pair of a raw projection medical image and a target medical image, wherein both include the same image content, the missed piece of information is compensated or even generated using a trained AI-based model. The trained AI-based model either directly generates the missed medical images or generates other types of data being appropriate for a comparison for adapting the medical image processing chain.

For that object, a method for generating a trained AI-based model for estimating a missing piece of information of a complete matching pair of medical image data related to a target flavor, preferably synthetic image data or representation data, according to the invention is also provided.

The method comprises the steps of:
- generating input data, wherein the input data comprise medical image data representing only a piece of information of a complete matching pair related to a target flavor,
- applying the input data to an AI-based model to be trained, wherein result data are generated,
- training the AI-based model based on the result data,
- providing the trained AI-based model.

The medical image data preferably comprise at least one of the following data sets:
- a target medical image,
- a raw projection medical image,
- an estimated medical image.

The result data may comprise information useful for achieving the missing piece of information, preferably synthetic image data or representation data, used for compensating the missing piece of information of the input data.

Advantageously, an AI-based model used for estimating missing information, preferably synthetic image data or representation data, can be flexibly adapted to a deliberate data basis of training data.

If a supervised training is to be carried out, the method for generating a trained AI-based model for estimating missing information of a piece of information of a complete matching pair of medical image data related to a target flavor, preferably synthetic image data or representation data, according to the invention, comprises the step of generating labelled input data including input data and validated result data. The labelled input data comprise only a piece of information of a complete matching pair of medical image data related to a target flavor and the validated result data comprise the missing information of the piece of information of a complete matching pair of medical image data related to a target flavor.

Preferably, the labelled input data comprise one of the following data sets:
- a target medical image as input data and a validated raw projection medical image as validated result data,
- a raw projection medical image as input data and a validated target medical image as validated result data,
- an estimated medical image or a target medical image as input data and representation data representing a flavor of the medical image as validated result data.

The above mentioned variant further comprises the steps of:
- applying the labelled input data to an AI-based model to be trained, wherein result data are generated,
- training the AI-based model based on the result data and the validated result data,
- providing the trained AI-based model.

As validated result data, a medical image comprising a predetermined flavor of the assigned medical image or representation data representing the flavor of the assigned medical image are to be understood. The training of the AI-based model can be implemented using a backpropagation algorithm or a cost function for adapting the AI-based model to the labelled input data. Using a supervised training comprises the advantage that the training data basis need not to be as extensive as it has to be in the variant of an unsupervised or self-supervised training. Further, the artificial neural network structure need not to be as complex as for the variant of an unsupervised or self-supervised training.

Alternatively, the method for generating a trained AI-based model for estimating a missing piece of information, preferably synthetic image data or representation data, according to the invention, can be implemented as a training for an unsupervised or self-supervised network, in particular a generative adversarial network. That means that the training data need not to be labelled at al. Hence, in the alternative variant, the training is performed by using unlabelled input data and the step of training the AI-based model comprises an unsupervised or self-supervised training step. Such a variant involves less effort compared to a supervised training using labelled training data.

A generative adversarial network consists of two artificial neural networks performing a zero-sum game. One of them creates candidates (the generator), the second neural network evaluates the candidates (the discriminator).

Typically, the generator maps from a vector of latent variables to the desired result space. For example, the latent variables inform about the style of a target medical image. The aim of the generator is to learn how to generate results according to a specific distribution. For example, the results comprise the synthetic raw projection medical image. The discriminator, on the other hand, is trained to distinguish the results of the generator from the data from the real, given distribution. The generator's objective function or cost function is then to produce results that the discriminator cannot distinguish. This should gradually adjust the generated distribution to the real distribution.

For that reason, a sufficiently large data set consisting of real unlabelled input data is required to train such a model. These unlabelled input data may comprise incomplete matching pairs of medical image data related to a target flavor and preferably data sets including a target medical image as input data and data sets including a raw projection medical image as input data.

These input data are used to train a discriminator until it reaches an acceptable level of accuracy. During subsequent training, a generator is given a random sample of a distribution of result data chosen from a previously defined range of latent variables. From this, the generator tries to generate a new distribution. This distribution is then presented to the discriminator, which tries to distinguish it from a real one. The weights of both models are independently improved by backpropagation, allowing the generator to create better distributions and the discriminator to better recognize them. Through this game, both models constantly improve each other, which, given sufficient training time, leads to generated distributions that cannot be distinguished from real ones.

The adaption device according to the invention comprises an input interface for receiving medical image data representing a piece of information of a complete matching pair related to a target flavor. The adaption device also comprises an estimation unit for generating an estimated medical image by applying a medical image processing chain to a raw projection medical image. Further, the adaption device according to the invention includes a comparison unit for determining a result of a comparison based on the estimated medical image and a target medical image. Furthermore, the adaption device according to the invention comprises an adaption unit for generating an adapted medical image processing chain by adapting the medical image processing chain based on the result of the comparison.

The adaption device according to the invention shares the advantages of the method for AI-assisted generating an adapted medical image processing chain according to the invention.

Some units or modules of the adaption device mentioned above, in particular the estimation unit, the comparison unit and the adaption unit, can be completely or partially realized as software modules running on a processor of a respective computing system, e.g. of a control device of a finding system or a medical imaging system. A realization largely in the form of software modules can have the advantage that applications already installed on an existing computing system can be updated, with relatively little effort, to install and run these units of the present application. The object of the invention is also achieved by a computer program product with a computer program or by a computer program that is directly loadable into the memory of a computing system, and which comprises program units to perform the steps of the inventive method for AI-assisted generating an adapted medical image processing chain and the steps of the method for generating a trained AI-based model for estimating synthetic image data or representation data, when the program is executed by the computing system.

In particular, the method for AI-assisted generating an adapted medical image processing chain may include the following steps executable by a computer program: the step of generating an estimated medical image, the step of determining a result of a comparison based on the estimated medical image and a target medical image and the step of generating an adapted medical image processing chain.

The method for AI-assisted generating an adapted medical image processing chain may also include the following steps executable by a computer program later described in detail: the step of generating a synthetic target medical image by applying a first trained AI-based model to the raw projection medical image or a synthetic raw projection medical image by applying a second trained AI-based model to the target medical image or first representation data representing a flavor of the target medical image by applying a third trained AI-based model to the target medical image.

The method for AI-assisted generating an adapted medical image processing chain may also include the following steps executable by a computer program later described in detail: the step of generating an estimated medical image by applying the medical image processing chain to the raw projection medical image or the synthetic raw projection medical image, the step of determining a result of a comparison between the estimated medical image and the target medical image or the synthetic target medical image or generating second representation data representing a flavor of the estimated medical image by applying the third trained AI-based model to the estimated medical image and determining a result of a comparison between the first representation data and the second representation data and the step of generating an adapted modelled medical image processing chain by adapting the medical image processing chain based on the result of the comparison.

Further, the method for generating a trained AI-based model for estimating synthetic image data or representation data may also include the following steps executable by a computer program: the step of generating input data, the step of applying the input data to an AI-based model to be trained, wherein result data are generated, and the step of training the AI-based model based on the result data.

In addition to the computer program, such a computer program product can also comprise further parts such as documentation and/or additional components, also hardware components such as a hardware key (dongle etc.) to facilitate access to the software.

A computer readable medium such as a memory stick, a hard-disk or other transportable or permanently-installed carrier can serve to transport and/or to store the executable parts of the computer program product so that these can be read from a processor unit of a computing system. A processor unit can comprise one or more microprocessors or their equivalents.

The dependent claims and the following description each contain particularly advantageous embodiments and developments of the invention. In particular, the claims of one claim category can also be further developed analogously to the dependent claims of another claim category. In addition, within the scope of the invention, the various features of different exemplary embodiments and claims can also be combined to form new exemplary embodiments.

In a variant of the method for AI-assisted generating an adapted medical image processing chain according to the invention, the automatic steps are automatically iteratively repeated using the adapted medical image processing chain of the third step as medical image processing chain in the first step. Advantageously, the adaption is improved with every iteration step such that an iterative approach to an optimized parameterization of the medical image processing chain is achieved. The adaption of the medical image processing chain based on a comparison based on the estimated medical image and the target medical image can be formulated by using an objective function, also called a loss or an error function, which indicates if the desired flavor is met. The parameter values of the parameters of the medical image processing chain are iteratively adjusted until the value of the objective function is as low as possible.

Preferably, the three steps of the method for AI-assisted generating an adapted medical image processing chain are automatically iteratively repeated, until a predetermined quality criteria or optimum criteria for the result of the comparison in the second step is achieved. Advantageously, the iteration is repeated, until the precision of the optimization achieves a predetermined level or an optimum level. Ideally, the medical image processing chain is differentiable and the parameter values of the parameters medical image processing chain can be adjusted by doing a stochastic gradient descent. Otherwise, an optimization can be performed based on numerical approaches.

In a variant of the method for AI-assisted generating an adapted medical image processing chain, wherein a matching pair of a raw projection medical image and a target medical image with the same content is missed and only a raw projection medical image is received and therefore available, a synthetic target medical image is generated by applying a first trained AI-based model to the raw projection medical image in advance. Further, the comparison in the second step is performed by comparing the estimated medical image with the synthetic target medical image being used as the target medical image. Advantageously, a matching pair of a raw projection medical image and a target medical image is artificially generated. The artificially generated matching pair can be used for adapting a medical image processing chain by comparing these different images including the same content. Advantageously, an adaption of a medical imaging processing chain is possible even if a matching pair of a raw projection medical image and a target medical image with the same content is not available.

In a further variant of the method for AI-assisted generating an adapted medical image processing chain, the received medical image data comprises a target medical image, but not a raw projection medical image. Hence, a synthetic raw projection medical image is generated by applying a second trained AI-based model to the target medical image. Typically, there is only one set of processed images from the radiologist with the desired style, but there are not matching unprocessed raw projection medical image including the same image content. Then, the generation of an estimated medical image in the first step is performed by applying the medical image processing chain to the synthetic raw projection medical image being used as the raw projection medical image and the comparison in second step is performed by comparing the estimated medical image with the target medical image. Advantageously, an adaption of a medical imaging processing chain is possible even if a target medical image of the desired flavor is available, but no matching raw projection medical image is available.

In an alternative variant of the method for AI-assisted generating an adapted medical image processing chain, a pair of an unprocessed raw projection medical image and a target medical image of the desired flavor is received and therefore available, but the content of the raw projection medical image is not the same as the content of the target medical image.

Therefore, first representation data representing a flavor of the target medical image by applying a third trained AI-based model to the target medical image are generated in advance.

Then, in the second step of determining a result, second representation data representing a flavor of the estimated medical image are generated by applying the third trained AI-based model to the estimated medical image and the result is determined by a comparison between the first representation data and the second representation data. Advantageously, the content of the pair of medical image data used for adapting the medical image processing chain does not need to be the same, which improves the flexibility of the adaption process.

Preferably, the first representation data and the second representation data comprise a unit vector and the number of the dimension of the unit vector is the same as the number of different possible flavors represented by the first representation data and the second representation data. Advantageously, a comparison between different representation data can be performed based on a norm of a difference between the two unit vectors representing the first representation data and the second representation data, wherein the result is normalized and therefore easily comparable with a normal.

Preferably, the result comprises a difference between the compared images or compared representation data and the predetermined quality criteria comprises a maximum allowed difference or norm of a difference between the first and second representation data or an indication for a minimized value of the difference or a minimized value of a norm of the difference. Advantageously, the minimum precision of the adaption of the medical image processing chain can be controlled and predetermined.

Particularly preferably, the adapted modelled medical image processing chain is achieved by generating an adapted parameter value by adapting a parameter value of at least one parameter of the medical image processing chain and the adapted medical image processing chain is achieved by taking over the adapted parameter value for the adapted medical image processing chain. Advantageously, the parameter values of the medical image processing chain can be automatically adapted and even a matching set of a pair of a raw projection medical image and a target medical image with the same content is not available.

In a variant of the method for generating an adapted medical image processing chain according to the invention, the first or second AI-based model is trained using an un- or self-supervised method.

Unsupervised learning refers to machine learning with no known target values and no reward from the environment. The learning algorithm or model tries to recognize patterns in the input data that deviate from structureless noise.

Preferably, the un- or self-supervised method comprises a generative adversarial network. A generative adversarial network (GAN for short) is a machine learning model capable of generating data. It consists of two competing artificial neural networks (ANN for short). One has the task of generating real-looking data, the other classifies the data as real or artificial. Through constant learning and many iteration steps, the generated data is getting better and better. A typical area of application is the creation of realistic-looking artificial images. Advantageously, synthetic raw projection medical images and synthetic target medical images can be generated very realistic. Prominent representatives based on GANs are described in Zhu, Jun-Yan, et al. "Unpaired image-to-image translation using cycle-consistent adversarial networks." Proceedings of the IEEE international conference on computer vision. 2017 and in Park, Taesung, et al. "Contrastive learning for unpaired image-to-image translation." European conference on computer vision. Springer, Cham, 2020.

It is also possible to "augment" different flavors with an image processing chain. In that way, there are matching pairs of raw projection medical images and processed target medical images. An artificial neural network can then be trained in an end-to-end fashion to learn to match flavoured target medical images back to raw projection medical images or vice versa. Diffusion models shown in Zhao, Min, et al. "Egsde: Unpaired image-to-image translation via energy-guided stochastic differential equations." arXiv preprint arXiv: 2207.06635 (2022) might prove especially suitable for this task. If the data augmentation is diverse enough, the network is then able to match unseen flavors from desired flavor back.

It is also possible to compare multiple medical images at once. The average of the differences can then be used to optimize the medical image processing chain. To train such a flavor representing network, a dataset of X-ray medical images with different flavors is necessary. Moreover, the information, which medical images have the same flavor, must be included. However, no matching pairs are needed. The flavor representing network has then to be trained to minimize the representation distance between two medical images of the same flavor and maximize the distance between two medical images with different flavors.

Depending on the number of different flavors, different training strategies are suitable. For a discrete number of flavors, a classification in a one hot encoder fashion is possible. A 1-of-n code, also known as one-hot coding, represents numbers in binary, usually for use in digital technology or computers. In that procedure, each flavor is represented by a unit vector in the direction of only one dimension, e.g. [0, 0, 1, 0]. In that example, we would have four different flavors, for each flavor the vector points in another direction. The network is trained to estimate these vectors. Ideally, unseen flavors match either one of the training flavors or lie between some flavors used for training, so that the represented flavor is still a unit vector and lies on a unit sphere, e.g. [0, 0.5, 0.866, 0].

Due to data augmentation, in that case flavor augmentation, an infinite number of flavors is possible. In that case, another loss function must be applied to train the flavor representing network. Contrastive learning, described in Chen, Ting, et al. "A simple framework for contrastive learning of visual representations." International conference on machine learning. PMLR, 2020, and siamese learning, described in Melekhov, Iaroslav, Juho Kannala, and Esa Rahtu. "Siamese network features for image matching." 2016 23rd international conference on pattern recognition (ICPR). IEEE, 2016, are two possible options.

Typically, in contrastive learning, the network get's a set of images for each training iteration. It then must choose which of these images belong together and which images do not belong together. Using the siamese loss, the network only gets two medical images and must decide if these two medical images belong together or not. Usually, both approaches are used, to train a network to represent the content of an image. In our case, the network must find the right flavors, which belong together and so it learns to represent the X-ray image flavor. In a contrastive or a siamese procedure, several activated last layers of the network serve as representations.

Hence, in an also preferred variant of the method for generating an adapted medical image processing chain of the invention, the representation data are compared and the number of different possible flavors is infinite and the first representation data and the second representation data comprise several activated last layers of an AI-based network which is based on contrastive learning or siamese learning. Advantageously, the scale of different flavors can be arbitrarily finely subdivided.

In a variant of the method for generating a trained AI-based model for estimating synthetic image data or representation data, augmented labelled input data are generated as input data by applying a modelled medical image processing chain to unlabelled input data. Advantageously, the training data basis can be extended to more different training data which increases the flexibility and robustness of the trained AI-based model.

In a preferred variant of the adaption device according to the invention, the adaption device comprises an input interface for receiving a medical image processing chain and one of the following image data:
- a raw projection medical image or
- a target medical image or
- a raw projection medical image and a target medical image, wherein the content of the raw projection medical image is not the same as the content of the target medical image.

As explained in detail, if a complete matching pair is provided, the adaption of the medical imaging processing chain can be achieved by a direct comparison between an estimated medical image generated based on the raw projection medical image and the target medical image of the matching pair. If one member of the matching pair is missed, the missed member can be generated using an AI-based model. If one member of the matching pair is missed, however a raw projection medical image and a target medical image including a different content are available, instead of a direct comparison, a comparison of the flavor of these images with different content can be performed for generating an adapted medical imaging processing chain for reconstruction of medical images with the desired flavor.

Also preferably, the adaption device according to the invention comprises a generation unit for generating at least one of the following types of data:
- a synthetic target medical image by applying a first trained AI-based model to the raw projection medical image or
- a synthetic raw projection medical image by applying a second trained AI-based model to the target medical image,
- first representation data representing a flavor of the target medical image by applying a third trained AI-based model to the target medical image.

Hence, the generation unit is used for replacing missed matching image data.

In a preferred variant of the adaption device according to the invention, the estimation unit is arranged for generating an estimated medical image by applying the medical image processing chain to the raw projection medical image or the synthetic raw projection medical image. Advantageously, the estimation unit is capable to complete the incomplete matching pair of medical image data related to a target flavor such the an adaption of the medical image processing chain to a desired flavor can be successfully performed.

Also preferred, the comparison unit of the adaption device is arranged for
- determining a result of a comparison between the estimated medical image and
   - the target medical image or
   - the synthetic target medical image or for
- generating second representation data representing a flavor of the estimated medical image by applying the third trained AI-based model to the estimated medical image and for determining a result of a comparison between the first representation data and the second representation data.

Advantageously, the type of comparison depends on the type of comparable data, in particular it depends thereon if the content of the estimated medical image and the target medical image is the same or not. Advantageously, the comparison unit is enabled to compare even different medical images including different image contents.

The invention is explained below with reference to the figures enclosed once again. The same components are provided with identical reference numbers in the various figures.
The figures are usually not to scale.
- FIG 1: shows a schematic view on two reconstructed volumes of the same breast scan showing two different flavors,
- FIG 2: shows a schematic view on a scan of the same phantom generated by different medical imaging systems,
- FIG 3: shows a schematic diagram illustrating a method for AI-assisted generating an adapted medical image processing chain according to a first embodiment of the invention,
- FIG 4: shows a schematic diagram illustrating a method for AI-assisted generating an adapted medical image processing chain according to a second embodiment of the invention,
- FIG 5: shows a flow chart diagram illustrating the method for AI-assisted generating an adapted medical image processing chain according to the first embodiment of the invention,
- FIG 6: shows a flow chart diagram illustrating the method for AI-assisted generating an adapted medical image processing chain according to the second embodiment of the invention,
- FIG 7: shows a flow chart diagram illustrating the method for generating a trained AI-based model for estimating a missing piece of information of a complete matching pair of medical image data related to a target flavor, in particular synthetic image data or representation data, according to an embodiment of the invention,
- FIG 8: shows a schematic view on an adaption device according to an embodiment of the invention.

In FIG 1, a schematic view 10 on two reconstructed volumes 10a, 10b of the same breast scan showing two different flavors is shown. As can be taken from FIG 1, the left volume 10a is "darker" than the right volume 10b. The characteristic property of "flavour" can be defined based on a determined set of different parameters. The type of these parameters and hence the definition of the "flavour" is different dependent on the manufacturer of the X-ray imaging system.

In FIG 2, a schematic view 20 on a scan of two different versions of images 20a, 20b of the same phantom generated by different medical imaging systems is shown. As can be taken from FIG 2, the left volume 20a is "darker" than the right volume 20b.

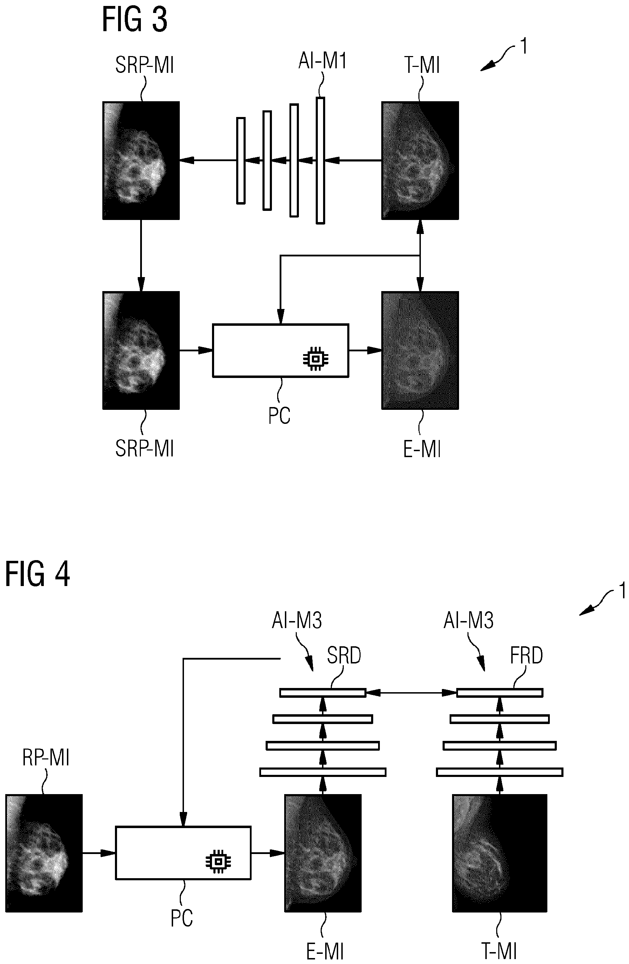

In FIG 3, a schematic diagram 1 illustrating a method for AI-assisted generating an adapted medical image processing chain A-PC according to a first embodiment of the invention is shown.

A first artificial neural network AI-M1 can be trained to generate matching medical images SRP-MI, T-MI. This can be achieved, by estimating matching unprocessed synthetic raw projection medical images SRP-MI to the target medical images T-MI with the desired flavor. This case is used in the optimization depicted in FIG 3.

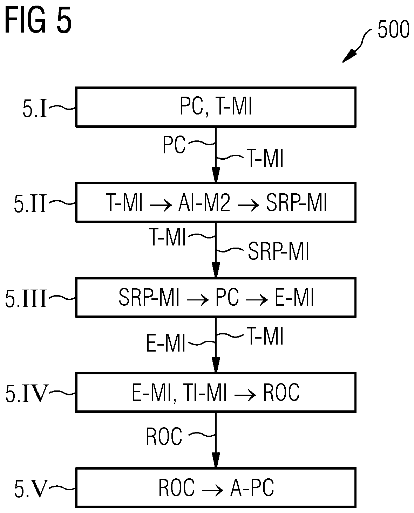

However, it is also possible, to estimate processed images i.e. synthetic target medical images to corresponding raw projection medical images SRP-MI using a second artificial neural network AI-M2 (shown in FIG 5). The generated synthetic target medical images should then have the same flavor as target medical images comprising the desired flavor. One way to train such a network are un- or self-supervised methods. This problem is often called "unpaired image-to-image translation" in literature. Having generated a matching pair of a raw projection medical image SRP-MI and an assigned target medical image T-MI, the medical image processing chain PC can be applied to the matching pair and an estimated medical image E-MI is generated based on the raw projection medical image SRP-MI of the matching pair. After that, a comparison between the estimated medical image E-MI and the target medical image T-MI is performed and the parameter values of the parameterized medical image processing chain PC are changed depending on the result of the comparison. The before-mentioned steps can be iteratively repeated, until an optimum of an adapted medical image processing chain PC, based on the result of the comparison, is achieved.

In FIG 4, a schematic diagram 1 illustrating a method for AI-assisted generating an adapted medical image processing chain A-PC according to a second embodiment of the invention is depicted.

As in the first embodiment illustrated in FIG 3, the medical image processing chain PC generates an estimated medical image E-MI based on a raw projection medical image RP-MI.

The idea underlying the second embodiment is that an objective function can be designed, which is able to compare the flavor of two medical images, even though these two medical images do not have the same content. A third AI-based model AI-M3 can be trained, which is able to represent the flavor of an estimated medical image E-MI or a target medical image T-MI without taking into account the different content of these images E-MI, T-MI. The representation data FRD, SRD related to the flavor of two medical images, i.e. the estimated medical image E-MI and the target medical image T-MI, can then be compared against each other. Hence, the generation of such representation data FRD, SRD enables the comparison of an estimated medical image E-MI, processed by the medical image processing chain PC, with a target medical image T-MI including the desired flavor and based thereon, an objective function can be generated, which can be used for an optimization of the medical image processing chain PC.

In FIG 5, a flow chart diagram 500 illustrating the method for AI-assisted generating an adapted medical image processing chain A-PC according to the first embodiment of the invention is depicted.

In step 5.I, a medical image processing chain PC is received and a target medical image T-MI is received.

In step 5.II, a synthetic raw projection medical image SRP-MI which comprises the same content as the received target medical image T-MI is generated by applying a second trained AI-based model AI-M2 to the target medical image T-MI received in step 5.I.

In step 5.III, an estimated medical image E-MI is generated by applying the medical image processing chain PC to the synthetic raw projection medical image SRP-MI, generated in step 5. II.

In step 5.IV, a result ROC of a comparison between the estimated medical image E-MI and the target medical image T-MI received in substep 5.I is determined.

In step 5.V, an adapted medical image processing chain A-PC is generated by adapting the medical image processing chain PC based on the result ROC of the comparison.

It has to be mentioned that the steps 5.III to 5.V are iteratively repeated, until a quality criteria is achieved. The achievement of the quality criteria is determined based on the comparison in step 5.IV. That means that the iteration is repeated, until the precision of the optimization achieves a predetermined level or an optimum level.

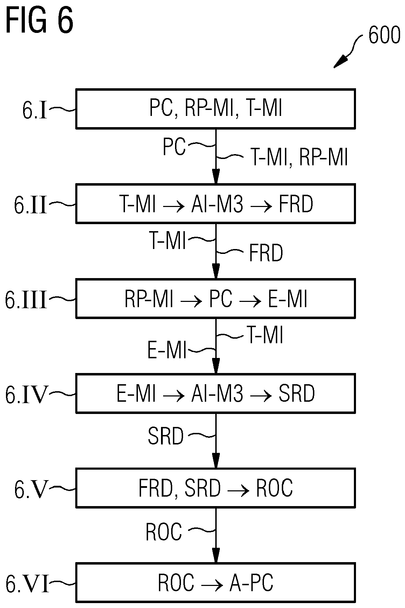

In FIG 6, a flow chart diagram 600 illustrating the method for AI-assisted generating an adapted medical image processing chain A-PC according to a second embodiment of the invention corresponding to the scheme shown in FIG 4, is presented.

In step 6.1, a medical image processing chain PC and a raw projection medical image RP-MI and a target medical image T-MI, wherein the content of the raw projection medical image RP-MI is not the same as the content of the target medical image T-MI, are received.

In step 6.II, first representation data FRD representing a flavor of the target medical image T-MI are generated by applying a third trained AI-based model AI-M3 to the target medical image T-MI received in Step 6.1.

In step 6.III, an estimated medical image E-MI is generated by applying the medical image processing chain PC to the raw projection medical image RP-MI received in step 6.1.

In step 6.IV, second representation data SRD representing a flavor of the estimated medical image E-MI are determined by applying the third trained AI-based model AI-M3 to the estimated medical image E-MI.

In step 6.V, a result ROC of a comparison between the first representation data FRD generated in step 6.II and the second representation data SRD is determined.

In step 6.VI, an adapted medical image processing chain A-PC is generated by adapting the medical image processing chain PC based on the result ROC of the comparison.

It has to be mentioned that the steps 6.III to 6.VI are iteratively repeated, until a quality criteria is achieved. The achievement of the quality criteria is determined based on the comparison in step 6.V.

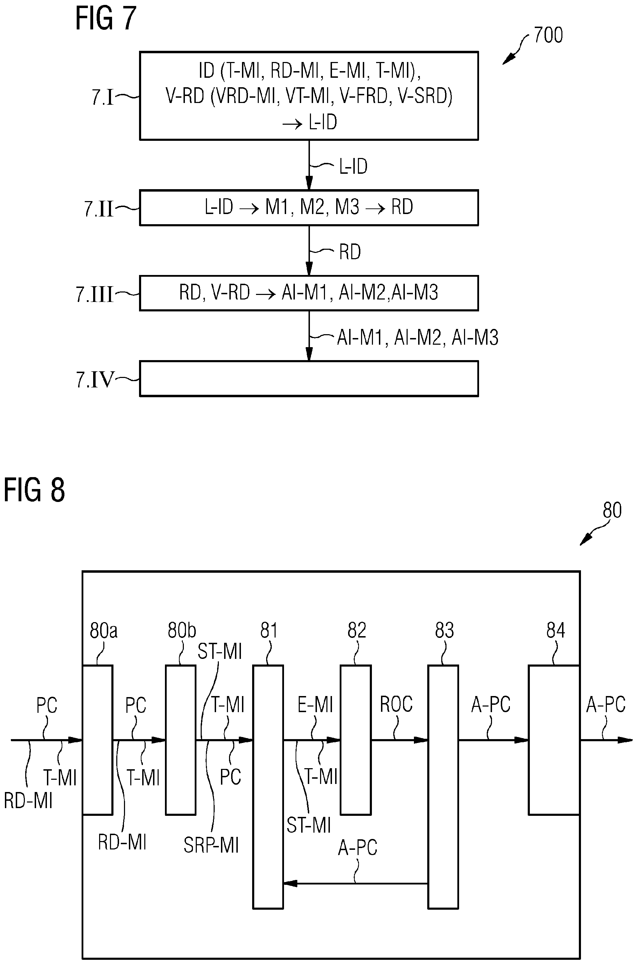

In FIG 7, a flow chart diagram 700 illustrating the method for generating a trained AI-based model AI-M1, AI-M2, AI-M3 for estimating a missing piece of information of a complete matching pair of medical image data related to a target flavor, in particular synthetic medical images SRP-MI or representation data FRD, SRD, according to an embodiment of the invention, is depicted.

In step 7.I, labelled input data L-ID including input data ID and validated result data V-RD are generated. The labelled input data L-ID comprise one of the following data sets:
- a target medical image T-MI as input data ID and a validated raw projection medical image VRP-MI as validated result data V-RD,
- a raw projection medical image RP-MI as input data ID and a validated target medical image VT-MI as validated result data V-RD,
- an estimated medical image E-MI or a target medical image T-MI as input data ID and validated representation data V-FRD, V-SRD representing a flavor of the medical image E-MI, T-MI as validated result data V-RD.

In step 7.II, the labelled input data L-ID are applied to an AI-based model M1, M2, M3 to be trained, wherein result data RD are generated.

In step 7.III, a trained AI-based model M1, M2, M3 is generated based on the result data RD and the validated result data V-RD.

In step 7.IV, the trained AI-based model AI-M1, AI-M2, AI-M3 is provided to a user.

In FIG 8, a schematic view on an adaption device 80 according to an embodiment of the invention is shown.

The adaption device 80 comprises an input interface 80a for receiving a medical image processing chain PC and in the variant shown in FIG 8, a target medical image T-MI or alternatively raw projection medical images RP-MI.

Further, the adaption device 80 includes a generation unit 80b for generating a synthetic raw projection medical image SRP-MI by applying a second trained AI-based model AI-M2 to the target medical image T-MI or alternatively synthetic target medical images ST-MI if only raw projection medical images RP-MI were received.

Part of the adaption device 80 is also an estimation unit 81 for generating an estimated medical image E-MI by applying the medical image processing chain PC to the synthetic raw projection medical image SRP-MI or alternatively to the raw projection medical image RP-MI.

The adaption device 80 also comprises a comparison unit 82 for determining a result ROC of a comparison between the estimated medical image E-MI and the target medical image T-MI or alternatively the synthetic target medical image ST-MI.

Further, the adaption device 80 includes an adaption unit 83 for generating an adapted medical image processing chain A-PC by adapting the medical image processing chain PC based on the result ROC of the comparison. In an iteration, the adaption unit 83 transmits the adapted medical image processing chain A-PC to the estimation unit 81 for generating an estimated medical image E-MI by applying the adapted medical image processing chain A-PC to the synthetic raw projection medical image SRP-MI or alternatively to the raw projection medical image RP-MI.

Furthermore, the adaption device 80 comprises an output interface 84 for outputting the adapted image processing chain A-PC after completing the iteration between the adaption device 83, the estimation unit 81 and the comparison unit 82.

The above descriptions are merely preferred embodiments of the present disclosure but not intended to limit the present disclosure, and any modifications, equivalent replacements, improvements, etc. made within the spirit and principle of the present disclosure should be included within the scope of protection of the present disclosure.

Further, the use of the undefined article "a" or "one" does not exclude that the referred features can also be present several times. Likewise, the term "unit" or "device" does not exclude that it consists of several components, which may also be spatially distributed. Furthermore, independent of the grammatical term usage, individuals with male, female or other gender identities are included within the term.

## Claims

1. Method for AI-assisted generating an adapted medical image processing chain (A-PC), comprising the automatic steps of:
i) receiving medical image data (RP-MI, T-MI) representing only a piece of information of a complete matching pair of medical image data related to a target flavor, wherein another piece of information (SRP-MI, ST-MI, FRD, SRD) of the complete matching pair is missing,
ii) generating an estimated medical image (E-MI) by applying the medical image processing chain (PC) to a raw projection medical image (RP-MI, SRP-MI) related to the received medical image data (RP-MI, T-MI),
iii) determining a result (ROC) of a comparison based on the estimated medical image (E-MI) and a target medical image (T-MI, ST-MI) related to the received medical image data (RP-MI, T-MI),
iv) generating an adapted medical image processing chain (A-PC) by adapting the medical image processing chain (PC) based on the result (ROC) of the comparison,
wherein the missing piece of information (SRP-MI, ST-MI, FRD, SRD) is generated based on the received medical image data (RP-MI, T-MI) using an AI-based model (AI-M1, AI-M2, AI-M3).

2. Method according to claim 1, wherein the steps ii) to iv) are automatically iteratively repeated using the adapted medical image processing chain (A-PC) of step iv) as the medical image processing chain (PC) in step ii).

3. Method according to any of the preceding claims, wherein the steps ii) to iv) are automatically iteratively repeated, until a predetermined quality criteria or optimum criteria for the result (ROC) of the comparison in step iii) is achieved.

4. Method according to any of the preceding claims, wherein
- the received medical image data (RP-MI, T-MI) comprises a raw projection medical image (RP-MI),
- the generation of the missing piece of information (SRP-MI, ST-MI, FRD, SRD) comprises the generation of a synthetic target medical image (ST-MI) by applying a first trained AI-based model (AI-M1) to the raw projection medical image (RP-MI),
- the comparison in step iii) is performed by comparing the estimated medical image (E-MI) with the synthetic target medical image (ST-MI) being used as the target medical image (T-MI).

5. Method according to any of the claim 1 to 3, wherein
- the received medical image data (RP-MI, T-MI) comprises a target medical image (T-MI),
- the generation of the missing piece of information (SRP-MI, ST-MI, FRD, SRD) comprises the generation of a synthetic raw projection medical image (SRP-MI) by applying a second trained AI-based model (AI-M2) to the target medical image (T-MI),
- the generation of an estimated medical image (E-MI) in step ii) is performed by applying the medical image processing chain (PC) to the synthetic raw projection medical image (SRP-MI) being used as the raw projection medical image (RP-MI),
- the comparison in step iii) is performed by comparing the estimated medical image (E-MI) with the target medical image (T-MI).

6. Method according to any of the claim 1 to 3, wherein
- the received medical image data (RP-MI, T-MI) comprise a raw projection medical image (RP-MI) and a target medical image (T-MI), wherein the content of the raw projection medical image (RP-MI) is not the same as the content of the target medical image (T-MI),
- the generation of the missing information (SRP-MI, ST-MI, FRD, SRD) comprises the generation of first representation data (FRD) representing a flavor of the target medical image (T-MI) in advance by applying a third trained AI-based model (AI-M3) to the target medical image (T-MI),
- the step iii) of determining a result (ROC) comprises:
- generating second representation data (SRD) representing a flavor of the estimated medical image (E-MI) by applying the third trained AI-based model (AI-M3) to the estimated medical image (E-MI) and
- determining the result (ROC) by a comparison between the first representation data (FRD) and the second representation data (SRD).

7. Method according claim 6 wherein
- the first representation data (FRD) and the second representation data (SRD) comprise a unit vector and
- the number of the dimension of the unit vector is the same as the number of different possible flavors represented by the first representation data (FRD) and the second representation data (SRD).

8. Method according to claim 7, wherein the result (ROC) of the comparison in step iii) comprises a difference between a first unit vector of the first representation data (FRD) and a second unit vector of the second representation data (SRD).

9. Method according to any of the claims 6 to 8, wherein the number of different possible flavors is infinite and the first representation data (FRD) and the second representation data (SRD) comprise several activated last layers of an AI-based network, which is based on contrastive learning or siamese learning.

10. Method for generating a trained AI-based model (AI-M1, AI-M2, AI-M3) for estimating a missing piece of information (SRP-MI, ST-MI, FRD, SRD) of a complete matching pair of medical image data related to a target flavor, comprising the steps of:
- generating input data (ID), wherein the input data (ID) comprise medical image data (RP-MI, T-MI) representing only a piece of information of a complete matching pair of medical image data related to a target flavor,
- applying the input data (ID) to an AI-based model (M1, M2, M3) to be trained, wherein result data (RD) are generated,
- training the AI-based model (M1, M2, M3) based on the result data (RD),
- providing the trained AI-based model (AI-M1, AI-M2, AI-M3).

11. Method according to claim 10, wherein the input data (ID) comprise labelled input data (L-ID), including input data (ID) and validated result data (V-RD), wherein the labelled input data (L-ID) comprise one of the following data sets:
- a target medical image (T-MI) as input data (ID) and a validated raw projection medical image (VRP-MI) as validated result data (V-RD),
- a raw projection medical image (RP-MI) as input data (ID) and a validated target medical image (T-MI) as validated result data (V-RD),
- an estimated medical image (E-MI) or target medical image (T-MI) as input data (ID) and validated representation data (V-FRD, V-SRD) representing a flavor of the medical image (E-MI, T-MI) as validated result data (V-RD)
and wherein the step of training the AI-based model (M1, M2, M3) comprises a training based on the result data (RD) and the validated result data (V-RD).

12. Method according to claim 10, wherein the input data (ID) are generated as unlabelled input data (ID) and the step of training the AI-based model (M1, M2, M3) comprises an unsupervised or self-supervised training step.

13. Adaption device (80), comprising:
- an input interface (80a) for receiving medical image data (RP-MI, T-MI) representing only a piece of information of a complete matching pair of medical image data related to a target flavor, wherein another piece of information (SRP-MI, ST-MI, FRD, SRD) of the complete matching pair is missing,
- an estimation unit (81) for generating an estimated medical image (E-MI) by applying a medical image processing chain (PC) to a raw projection medical image (RP-MI, SRP-MI) related to the received medical image data (RP-MI, T-MI),
- a comparison unit (82) for determining a result (ROC) of a comparison based on the estimated medical image (E-MI) and a target medical image (T-MI) related to the received medical image data (RP-MI, T-MI),
- an adaption unit (83) for generating an adapted medical image processing chain (A-PC) by adapting the medical image processing chain (PC) based on the result (ROC) of the comparison,
wherein the missing piece of information (SRP-MI, ST-MI, FRD, SRD) is generated based on the received medical image data (RP-MI, T-MI) using an AI-based model (AI-M1, AI-M2, AI-M3).

14. A Computer program product comprising instructions which, when the program is executed by a computer, cause the computer to carry out the steps of the method for AI-assisted generating an adapted medical image processing chain (A-PC) according to any of the claims 1 to 9 or the steps of the method for generating a trained AI-based model (AI-M1, AI-M2, AIM3) for estimating a missing piece of information (SRP-MI, ST-MI, FRD, SRD) of a complete matching pair of medical image data related to a target flavor according to any of the claims 10 to 12.

15. A computer program comprising instructions which, when executed by a computer, cause the computer to carry out the steps of the method for AI-assisted generating an adapted medical image processing chain (A-PC) according to any of claims 1 to 9 or the steps of the method for generating a trained AI-based model (AI-M1, AI-M2, AI-M3) for estimating a missing piece of information (SRP-MI, ST-MI, FRD, SRD) of a complete matching pair of medical image data related to a target flavor according to any of the claims 10 to 12.
